(19) Europäisches Patentamt
European Patent Office
Office européen des brevets

(11) Publication number : **0 514 036 A2**

(12) **EUROPEAN PATENT APPLICATION**

(21) Application number : **92303814.5**

(22) Date of filing : **28.04.92**

(51) Int. Cl.$^5$ : **C07D 333/32,** C07D 409/04, C07D 473/40, C07F 7/18, A61K 31/505, A61K 31/52

(30) Priority : **29.04.91 GB 9109186**

(43) Date of publication of application : **19.11.92 Bulletin 92/47**

(84) Designated Contracting States : **AT BE CH DE DK ES FR GB GR IT LI LU MC NL PT SE**

(71) Applicant : **THE WELLCOME FOUNDATION LIMITED**
**Unicorn House 160 Euston Road**
**London NW1 2BP (GB)**

(72) Inventor : **Young, Robert John**
**Langley Court**
**Beckenham, Kent BR3 3BS (GB)**
Inventor : **Miller, John Allen**
**Langley Court**
**Beckenham, Kent BR3 3BS (GB)**

(74) Representative : **Garrett, Michael et al**
**The Wellcome Research Laboratories Group Patents and Agreements Langley Court**
**Beckenham Kent BR3 3BS (GB)**

(54) **Preparation of thionucleosides.**

(57) The invention relates to the preparation of homochiral thiolactones which have application in the synthesis of 3′- and 4′- thionucleosides. Substituents on the thiolactone can be used to influence the configuration of the C-1′ position in the final nucleoside. Configuration at the C-4′ position is controlled by use of the appropriate homochiral glycidol as starting material in the synthesis of the thiolactone. This process also offers the possibility of introducing substituents diastereoselectively in the C-2′ and C-3′ positions.

EP 0 514 036 A2

The present invention relates to the preparation of homochiral thiolactones which have application in the synthesis of 3′- and 4′-thionucleosides.

A number of thionucleosides have been shown to be useful in medical therapy, particularly in the treatment or prophylaxis of viral infections. Thus it is known from Secrist et al, J.Med.Chem., 1992, 35, 533-538 that certain 4′-thionucleosides, particularly the nucleoside of formula (I)

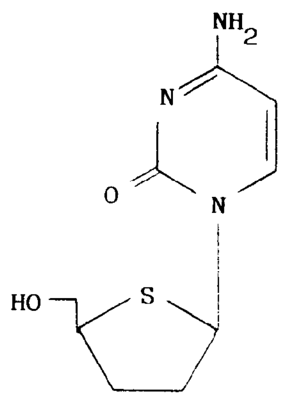

(I)

have potent anti-viral activity against Human Immunodeficiency Virus (HIV). Reference can also be made to the 4′,3′-oxathianucleosides of EP-A-382,526 which show activity against HIV. Other antiviral 4′-thionucleosides which are described in EP-A-409,575 show activity against Herpes viruses.

Nucleosides are chiral molecules and it is generally desirable to be able to synthesise them with a particular configuration at each stereogenic centre, especially at the C-4′ and C-1′ positions. Thionucleosides are generally difficult to prepare and, for those few compounds that have so far been made, a long synthesis involving many individual steps has been required (see, for example, Fu and Bobek, Nucleic Acid Chemistry, Vol. 1, Eds. Townsend and Tipson, page 317 and Secrist et al, reference as above.

The present invention relates to thiolactone intermediates which have been found to be useful in the synthesis of antiviral thionucleosides and which can be produced with both high enantiomeric and high diastereomeric selectivity thereby enhancing the enantiospecific synthesis of a wide range of 3′- and 4′-thionucleosides.

According to one aspect, the present invention provides a process for the preparation of γ-thiolactones of formula (II)

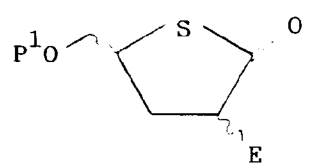

(II)

in which $P^1$ is a protecting group or hydrogen, and E is an anion stabilising group, which comprises reacting a thiirane of formula (III)

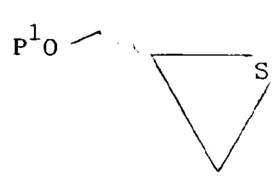

(III)

wherein $P^1$ is as defined above, with an anion derived from an α-substituted ester of formula (IV)

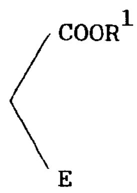

(IV)

where $R^1$ is $C_{1-6}$ alkyl, $C_{1-6}$ cycloalkyl or aryl and E is as defined above, or with a protected variant thereof; and, when a compound of formula (II) in which $P^1$ is a protecting group is prepared, optionally removing said protecting group to prepare a compound of formula (II) in which $P^1$ is hydrogen.

This process provides a simple and convenient route which enables the γ-thiolactones of formula (II) to be produced from readily available starting materials in a single process stage. Ring expansion of epoxides by anions of α-substituted esters to form γ-lactones are well known in the literature (see, for example, E. G. Lewars in "Comprehensive Heterocyclic Chemistry", Ed. W. Lwowski, Vol. 7, Chapter 5.05, p. 112; G. V. Boyd in Patai's "The Chemistry of Acid Derivatives, Part 1", 1979, Chapter 8, page 500).

Attempts to prepare γ-thiolactones by analogous ring expansion of thiiranes have previously been unsuccessful, resulting instead, in polymerisation of the thiirane. Related reactions of thiiranes are already known for achieving ring expansion to a five membered ring in which the sulphur anion is trapped by a nitrile group, e.g. reaction of the thiirane with the anion from ethyl cyanoacetate to give a 2-imino-3-ethoxycarbonyl-4,5-dihydrothiophene (Snyder and Alexander, J. Amer. Chem. Soc., 1948, 70, 217) or the anion from malononitrile to give 2-amino-3-cyano-4,5-dihydrothiophene (Yamagata et al, Chem. Pharm. Bull., 1982, 30, 4396).

The anion stabilising group E in compounds of formulae (IV) and (II) may be for example, an electron withdrawing group, such as a carboalkoxy eg. carbo-$C_{1-6}$alkoxy group, or an organo (eg. $C_{1-6}$alkyl or aryl such as phenyl), -sulphenyl, -sulphinyl, -sulphonyl or -selenyl.

According to an alternative embodiment of the invention applicable to the synthesis of 3′-thionucleosides and referred to in more detail below, the group E may be a radical derived from an optionally substituted purine or pyrimidine base such as the group B in formula (VI) below.

Suitable protecting groups $P^1$ include groups such that $P^1O$- is an ether group, e.g. a silyl ether group (such as tertbutyldiphenylsilyl ether or tertbutyldimethylsilyl ether), a straight or branched chain alkyl ether group, a cyclic ether group (such as tetrahydropyran-2-yl ether) or an optionally substituted aryl ether group (such as benzyl ether, trityl ether or benzhydryl ether). The group $P^1O$- can also be an ester group e.g. wherein $P^1$ is QC=O where Q is alkyl (such as methyl), cycloalkyl or an optionally substituted aryl.

The protecting group $P^1$ in the thiirane of formula (III) may be derived from precursors (eg. the glycidyl ether or glycidyl ester as described below) used to prepare the thiirane and may be retained in the synthesis of the γ-thiolactone of formula (II). The same protecting group will generally also be retained in the subsequent synthesis of a thionucleoside using the γ-thiolactone as an intermediate and will generally be cleaved only after the nucleoside bond has been formed and other manipulations have been executed. The protecting group $P^1$ is preferably a relatively bulky group which has important implications in influencing the stereochemical placement of other ligands around the thiosugar ring as will be explained in more detail below.

The ring expansion reaction can be carried out using a substantially homochiral thiirane of formula (III) without affecting the configuration at the stereogenic centre in the molecule, thereby leading to a γ-thiolactone of formula (II) which is substantially homochiral at the C-4 position. Suitable reaction conditions may be needed in any particular case to ensure that the desired ring expansion reaction takes place rather than some competing reaction such as polymerisation of the thiirane. In order to generate the anion of the compound of formula (IV), the latter may be treated with a base eg. sodium bis (trimethyldisilyl)amide, prior to reaction with the compound of formula (III). For example, when the protecting group $P^1$ is a silyl ether group such as tertbutyldiphenylsilyl and the compound of formula (IV) is dimethyl malonate, it has been found to be advantageous to add the thiirane to the preformed malonate anion in a suitable solvent such as tetrahydrofuran, with the concentration of the reactants being adjusted to ensure that the ring expansion reaction predominates.

According to a further aspect the present invention provides a γ-thiolactone of formula (II) as defined above in substantially homochiral form.

References herein to compounds being "in substantially homochiral form", denote compounds which have a single enantiomeric form and which are generally associated with less than 10% w/w, preferably less than 5% w/w, of the other enantiomer. These levels of enantiomeric purity, as described below, are generally governed by the enantiomeric purity of the starting commercial glycidols.

The present invention includes a process for the prepartion of a thiirane of formula (III) as defined above,

which comprises reacting a glycidol of formula (V) as defined below, with a suitable reagent such as thiourea.

It is known that thiiranes can be synthesised from the corresponding epoxides (see D. C. Dittmer in "Comprehensive Heterocyclic Chemistry", Ed. W. Lwowski, Vol. 7, Chapter 5.06, pages 178-179). The most suitable conditions for synthesis of the thiirane of formula (III) are those which allow a substantially homochiral thiirane to be prepared from a substantially homochiral glycidol, with inversion at the stereogenic centre. This is best achieved in the present case using thiourea as the source of the thiirane sulphur atom (see for example C-H. Wong et al, J. Org. Chem., 1990, 55, 4897).

Glycidols are commercially available, for example from Aldrich Chemical Company; glycidol, and a number of glycidyl ethers and glycidyl esters are available in their substantially homochiral (R)- and (S)- forms. Hereinafter, the (S)- and (R)- forms of glycidol and its derivatives are defined, as shown in formulae (Va) and (Vb), according to the convention referred to by Hanson, Chemical Reviews, 1991, 91 (4). The above methodology of Wong has been applied in accordance with the present invention to both racemic and substantially homochiral derivatives of a glycidol of formula (V)

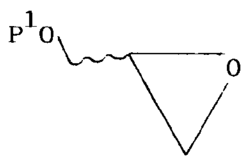

(V)

wherein $P^1$ is as defined above, since homochirality can be retained under the conditions for thiirane synthesis and ring expansion. Thus the (S)-enantiomer of glycidol (Va; $P^1$ = H)

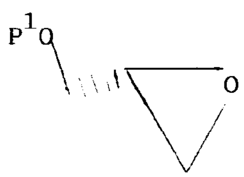

(Va)

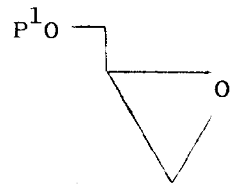

(Vb)

can be converted to a hydroxyl protected form of the (R)-enantiomer of the thiirane (III), hence into a thiolactone (II) which is substantially homochiral at the C-4 position, and subsequently, as described in more detail below, into a 4′-thionucleoside with only the natural D-configuration at the C-4′ position. As will also be described in more detail below, the (R)-enantiomer of glycidol (Vb; $R^1$ = H) can be converted to a hydroxyl protected form of the (S)-enantiomer of the thiirane (III), and subsequently into a 3′-thionucleoside of D-configuration at the C-4′ position.

The γ-thiolactones of formula (II) with the 4(S)-absolute configuration, i.e. compounds of the formula (IIA)

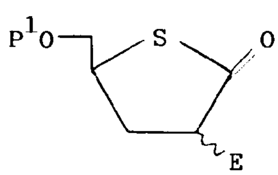

(IIA)

wherein $P^1$ and E are as defined above, are particularly useful as intermediates in the synthesis of 4′-thionucleosides of formula (VI)

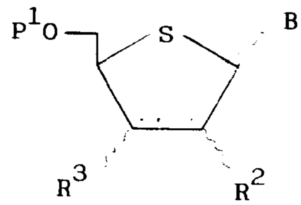

(VI)

wherein

$P^1$ is as defined above, B is a radical derived from an optionally substituted purine or pyrimidine base, and $R^2$ and $R^3$ are substituents on the thiosugar moiety, for example:

$R^2$ is hydrogen and $R^3$ is H, F, OH, $CH_2OH$ or $N_3$;

$R^2$ is OH or $CH_2OH$ and $R^3$ is H, OH or F;

$R^2$ is F and $R^3$ is H or OH; or

$R^2$ and $R^3$ together form a double bond.

The thionucleosides of formula (VI) are of particular interest in that the configuration at the C-4′ position is analogous to that in natural nucleosides. The relative configuration of $R^2$ and $R^3$ can be varied. Particular examples of this configuration are those derived from 4-thioribose, 4-thioarabinose, 4-thio-2-deoxyribose, 4-thio-2′,3′-dideoxyribose, 4-thio-2′,3′-didehydro-2′,3′-dideoxyribose, and substituted variants thereof.

Examples of the pyrimidine base from which the radical B in formula (VI) may be derived include compounds of formula (VII)

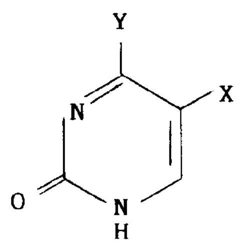

(VII)

where

Y is -OH or $-NH_2$; and

X is hydrogen, halogen, $C_{1-6}$alkyl, halo$C_{1-6}$alkyl, $C_{2-6}$alkenyl, halo$C_{2\ 6}$alkenyl, $C_{2-6}$alkynyl or $C_{1-3}$alkoxy$C_{1-6}$alkyl.

Alternatively, the group X in the compound of formula (VII) may be a group which can be converted into a group as defined above for X in a subsequent reaction after formation of the nucleoside bond. However, in the case of pyrimidine bases, it is generally preferred to carry out any manipulation to the group X prior to nucleoside bond formation.

Particular examples of compounds of formula (VII) are:

5-iodouracil;
5-ethyluracil;
5-fluorocytosine;
thymine;
5-ethynyluracil;
E-5-(2-bromovinyl)uracil;
cytosine;
5-(2-chloroethyl)uracil;
E-5-(1-propenyl)uracil; and
5-prop-1-ynyluracil.

Particular examples of purine bases from which the radical B in formula (VI) may be derived include:

guanine;
adenine;
2-amino-6-chloropurine; and

6-chloropurine.

In the case of purine bases, manipulation of the substitution on the purine ring preferably takes place after formation of the nucleoside bond. For example, use of 6-chloropurine allows access to a wide range of substituents at the 6- position on the purine ring.

As noted above, the γ-thiolactones of formula (IIA) are particularly useful as intermediates in the synthesis of 4′-thionucleosides. However, the compounds of formula (IIA) may be subjected to various chemical manipulations prior to formation of the nucleoside bond, for example (i) elimination or modification of the group E; (ii) replacement of the group E by another group which may have further chemical and/or stereochemical implications in the subsequent synthesis of the 4′-thionucleoside; or (iii) introduction of desired substituents in the thiosugar moiety of the 4′-thionucleoside to be produced, e.g. to produce a γ-thiolactone of formula (IX)

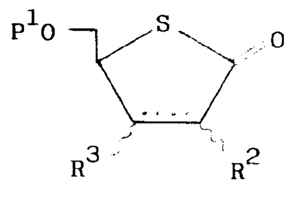

(IX)

wherein $P^1$, $R^2$ and $R^3$ are as defined above.

According to a further aspect the present invention provides a compound of formula (IX) as defined above in substantially homochiral form.

Certain of the groups represented by E can be eliminated from a γ-thiolactone of formula (IIA) by conventional methods, for example, when E is $-CO_2Me$, a compound of formula (IX) can be formed in which $R^2$ and $R^3$ are both hydrogen, for example, using Krapcho's method (Krapcho, Synthesis, 1982, 805 and 893).

The resulting compound of formula (IX) in which $R^2$ and $R^3$ are hydrogen can be converted into corresponding compounds of formula (IX) in which $R^2$ and $R^3$ are a variety of groups, as described in more detail below, by diastereoselective functionalisation of an enolate or silylenol ether of the former compound of formula (IX), ie. to produce a single diastereomer of formula (IX) which is associated with less than 20% and preferably less than 10% of any other diastereomers. The enolate can be formed by treatment of a compound of formula (IX) in which $R^2$ and $R^3$ are hydrogen with an appropriate base eg. Lithium hexamethyldisilazide, and optionally converted to the silylenol ether by subsequent treatment with a silylhalide eg. trimethylsilylchloride.

Compounds of formula (IX) in which $R^2$ is phenylsulphenyl, phenylsulphinyl or phenylsulphonyl can be obtained by reacting the enolate or silylenol ether derived from the corresponding compound of formula (IX) in which $R^2$ and $R^3$ are both hydrogen with phenylsulphenyl chloride, phenylsulphinyl chloride or phenylsulphonyl chloride. Compounds of formula (IX) in which $R^2$ is phenylselenyl can be prepared by reaction of the corresponding enolate or silylenol ether with phenylselenyl bromide (Chu et al, J. Org. Chem., 1990 55, 1418).

Compounds of formula (IIA) in which E is phenylsulphenyl, phenylsulphinyl, phenylsulphonyl or phenylselenyl are accessible by using a starting material of formula (IV) in which E is the corresponding group.

The compound of formula (IX) or (IIA) in which respectively $R^2$ or E is phenylselenyl can be converted into a compound of formula (IX) in which $R^2$ and $R^3$ together form a double bond by elimination of the selenium ligand via a selenoxide under oxidising conditions which are capable of oxidising selenium without oxidising sulphur, e.g. by treatment with m-chloroperbenzoic acid in dichloromethane at -20°C (Toru et al, Tetrahedron Letters, 1986, 27, 1583).

The compound of formula (IX) in which $R^2$ and $R^3$ together form a double bond can be used to introduce oxygen-based or other functionality into the 2′- and 3′-positions of compounds of formula (IX).

A fluorine atom in the α-configuration can be added to the 2-position (substituent $R^2$ in formula (IX)) by reaction of the enolate or silylenol ether derived from the corresponding compound of formula (IX) in which $R^2$ and $R^3$ are both hydrogen, with a suitable electrophilic fluorinating agent such as 1-fluoro-2,4,6-trimethylpyridinium triflate (Ihara et al, J. Chem. Soc. Perkin Trans. 1, 1990, 2357) or N-Fluoro, N-chloromethyl triethylenediamine bis (tetrafluoroborate). A bromine atom can be added in the same way in the α-configuration by reaction of the enolate or silylenol ether with elemental bromine (Kiesewetter and Margaratha, Helv. Chim. Acta., 1985, 68, 2350). HBr can be eliminated from a 2-α-bromo-lactone of formula (IX) by reaction with a non-nucleophilic base such as 1,8-diazabicyclo[5.4.0]- undec-7-ene to yield the compound of formula (IX) in which $R^2$ and $R^3$ together form a double bond.

The thiolactone of formula (IX) in which $R^2$ and $R^3$ are hydrogen may be hydroxylated under basic conditions to give a 2-α-hydroxylactone of formula (IX) using, for example, 3-phenyl-2-phenylsulphonyl oxaziridine (Vishwakarma et al, Org. Syn., 1988, 66, 203; Davis et al, J. Org. Chem., 1984, 49, 3241). Reaction of a 2-α-hydroxylactone of formula (IX) with diethylaminosulphurtrifluoride takes place with inversion leading to a 2-β-fluorolactone of formula (IX).

It is preferred that the group $R^2$ be added to the thiosugar ring of formula (IX) in a stereospecific manner leading to substantially only the epimer having the 2-α-configuration. As noted above in cases where the protecting group $P^1$ is a relatively bulky group, this may influence the introduction of the group $R^2$ in the desired manner. However, if the group $R^2$, for example a sulphur or selenium containing ligand such as -SPh or -SePh, is introduced into the 2-position of the thiosugar ring in a non-stereospecific way, then it is also possible to separate the mixture of the α- and β-epimers thus formed using chromatography, for example by use of an appropriate solvent gradient on a flash column. Treatment of the 2-β-epimer with a base, such as diethylamine or 1,8-diazabicyclo[5.4.0]undec-7-ene, followed by separation, provides further of the 2-α-epimer (Chu et al., J. Org. Chem., 1990, 55 1418) as a result of the influence of the bulky group $P^1$.

In order to convert the γ-thiolactones of formulae (II) and (IX) into a 4′-thionucleoside, the carbonyl group may first be converted into a suitable leaving group and the resulting compound is then reacted with an appropriate purine or pyrimidine base.

Thus, for example, a compound of formula (IIA) or formula (IX) is converted respectively to a compound of formula (IIA)L or (IX)L

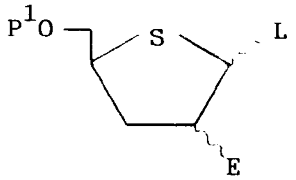
(IIA)L

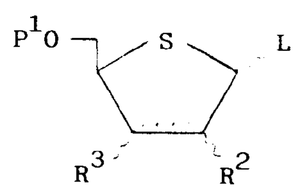
(IX)L

wherein $P^1$, E, $R^2$ and $R^3$ are as defined above and L is a leaving group, for example, an acyloxy group such as $C_{1-4}$alkanoyloxy, for instance, acetoxy; an organosulphonyloxy group such as methanesulphonyloxy or p-toluene-sulphonyloxy; an alkoxy group such as methoxy; or a halogen, such as chlorine. This conversion can be effected, for example, by reducing the starting compound of formula (IIA) or (IX) to convert the carbonyl group to a hydroxyl group, eg. by treatment with diisobutylaluminiumhydride, and then esterification, etherification or halogenation of the resulting thiolactol. The resulting compound of formula (IIA)L or (IX)L can then be reacted with an optionally protected pyrimidine or purine base to form the corresponding nucleoside.

According to the invention, the formation of a nucleoside bond can be carried out in a manner which leads to a 4′-thionucleoside substantially in the form of a single anomer. The γ-lactone intermediate for such a procedure is a compound of formula (IIA) in which the substituent E in the 2-position is in the α-configuration and may be a directing group such that the resulting nucleoside is largely in the β-configuration, i.e. the γ-lactone is a compound of formula (IIB)

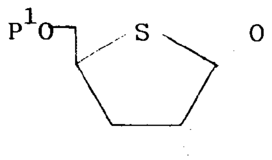
(IIB)

where Z is the directing group and $P^1$ is as defined above. In appropriate cases, the group Z can be eliminated following formation of the nucleoside bond.

The γ-thiolactone of formula (IIB) may be prepared from a compound of formula (IX) by introducing the directing group Z in the orientation as shown in formula (IIB). In general the group Z directs the incoming base into the desired stereochemical configuration. In this connection, reference can be made to Wilson & Liotta, Tetrahedron Letters, 1990, 31, 1815 for sulphenyl groups and Chu et al, J. Org. Chem., 1990, 55, 1418 for selenyl groups. The preferred group Z is phenylselenyl and this group can be introduced into a compound of formula (IX) in the manner described above with the orientation influenced by the protecting group $P^1$.

The compound of formula (IIB) can be converted into the mixture of anomers of the thiolactol of formula (XII)

(XII)

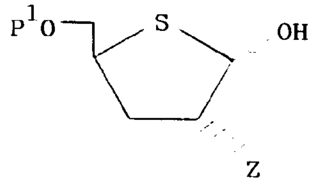

by reduction of the carbonyl group, for example, using conventional reagents such as diisobutylaluminium hydride.

The compound of formula (XII) can then be converted into a compound of formula (X)

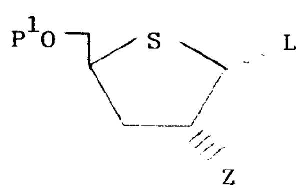

(X)

where L, Z and $P^1$ are as defined above, as described for the preparation of compounds of formulae (IIA)L and (IX)L, for example by acylation under standard conditions (e.g. acetic anhydride with pyridine) to produce the compound in which L is an acyloxy group, e.g. acetate.

The present invention further provides a process for the preparation of a 4'-thionucleoside of formula (VIA), (VI) or (XI)

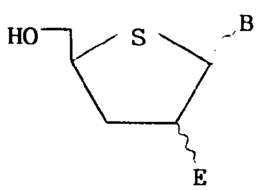

(VIA)

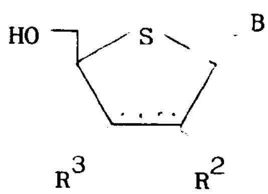

(VI)

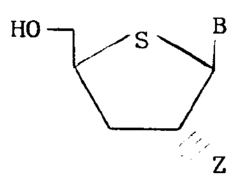

(XI)

wherein B, E, $R^2$, $R^3$ and Z are as defined above, which comprises reacting an optionally protected pyrimidine or purine base of formula B-H with respectively a compound of formula (IIA)L, (IX)L or (X) as defined above and subsequently removing any protecting group(s).

The base will generally be used in a form in which any functional groups are protected, for example by coupling according to the method of Vorbruggen (Chem. Ber., 1981, 114, 1234). For example, where the base is thymine, such a protected derivative will be a compound of formula (VIII)

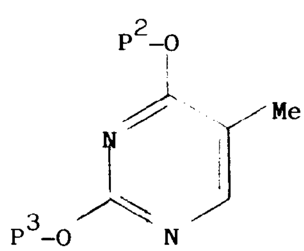

(VIII)

where $P^2$ and $P^3$, which may be the same or different, are suitable protecting groups, such as ether or silylether protecting groups, for example trimethylsilyl.

The reaction of the compound of formula (IIA)L, (IX)L or (X) with the base may be carried out for example in the presence of nonafluorobutane sulphonic acid or a Lewis acid catalyst, e.g. tin (IV) chloride, a mercury (II) salt or trimethylsilyl triflate. The reaction can be carried out, for example, at a temperature of from 0°C to room temperature in a suitable solvent such as acetonitrile or a chloroalkane.

The group Z may optionally be eliminated from the 4′-thionucleoside of formula (XI) following formation of the nucleoside bond. For example Z as phenylselenyl can be eliminated, i.e. a compound of formula (XI) in which Z is phenylselenyl can be converted into the corresponding compound of formula (VI) in which $R^2$ and $R^3$ are both hydrogen, under reducing conditions, for example using tributyltin hydride and triethyl borane (see Nozaki et al, Tetrahedron Letters, 1988, 29, 6125). The group Z can also be eliminated under oxidising conditions in the manner described above for the corresponding reaction on the compound of formula (II) in which E is phenylselenyl. Other substituents $R^2$ and $R^3$ can be introduced in the thiosugar ring following nucleoside bond formation in a similar manner to that described above for corresponding reactions in which a γ-thiolactone of formula (IIA) is converted to a γ-thiolactone of formula (IX) except for those reactions that require conjugate addition.

Reduction of a compound of formula (IIA) in which E is $-CO_2Me$ with diisobutyl aluminium hydride under standard conditions, affords a compound of formula (XIII)

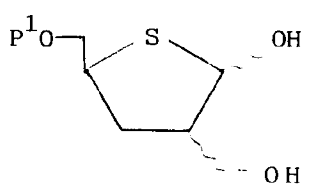

(XIII)

as a mixture of 2- α/β epimers which can be separated by chromatography. Compounds of formula (XIII) can be converted to 4′-thionucleosides by methods analogous to those described for compound (XII).

According to another embodiment of the invention, the reaction of the compound of formula (IIA)L or (IX)L with the base to form the nucleoside bond can be carried out in a manner which does not influence the orientation at the C-1′ position and thus leads to the 4′-thionucleoside as a mixture of anomers. Thus, the carbonyl group in a γ-thiolactone of formula (IIA) or (IX) may be converted into a suitable leaving group, for example an acyl group, in the manner described above. The resulting compound is then reacted with the appropriate purine or pyrimidine base, again in the manner described above; however, in the absence of a directing group Z to influence the orientation at the C-1′, the resulting 4′-thionucleoside will be produced as a mixture of anomers. If single anomers are required then the mixture can be separated by conventional techniques, for example the use of an appropriate solvent gradient on a flash column.

The γ-thiolactones of formula (II) with the 4(R)-absolute configuration, i.e. compounds of the formula (IIC)

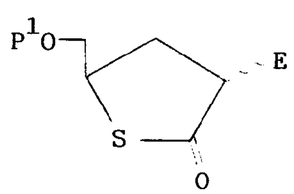

(IIC)

wherein $P^1$ and E are as defined above are particularly suitable for the synthesis of 3′-thionucleosides of formula (XIV)

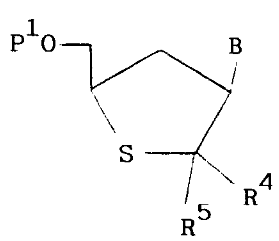

(XIV)

wherein $P^1$ and B are as defined above and $R^4$ and $R^5$ are suitable substituents on the thiosugar moiety, for example:

$R^4$ and $R^5$ both represent hydrogen;

$R^4$ and $R^5$ taken together represent =O;

$R^4$ is hydrogen and $R^5$ is -OH or a protected hydroxyl group;

or

$R^4$ is -OH or a protected hydroxyl group and $R^5$ is hydrogen.

The protecting group $P^1$ and the base B can be, for example as described above and $P^1$ is preferably tert-butyldiphenylsilyl. Suitable hydroxyl protecting groups are as described above with the size of the protecting group again being of importance.

Compounds of formula (XIV) can be made from the thiolactone (IID)

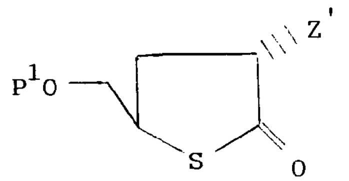

(IID)

where Z′ is a leaving group such as bromo, or an alkyl- or arylsulphonyl ester. The structures (IIB) and (IID) as depicted above are enantiomeric.

The compound of formula (IID) in which Z′ is bromine or in which Z′ is hydroxyl may be prepared in the same way as described above for the enantiomeric compounds (the 2-α-bromolactone and the 2-α-hydroxylactone derived from formula (IIB)). The resulting compound of formula (IID) in which Z′ is hydroxy may be converted into the corresponding sulphonyl ester under standard conditions (e.g. methanesulphonyl chloride with pyridine).

The nucleoside of formula (XIV) in which $R^4$ and $R^5$ taken together represent =0 may be prepared by reaction of the compound of formula (IID) with an unprotected base B under appropriate basic conditions e.g. potassium carbonate and dimethylsulphoxide (Jones et al, Tetrahedron Letters, 1991, 32, 247). The carbonyl group can be reduced to give an anomeric mixture of compounds of formula (XIV) in which one of $R^4$ and $R^5$ is hydrogen and the other is hydroxyl by reduction under standard conditions, e.g. using diisobutylaluminium hydride.

As mentioned above it is also possible for the group E in the compound of formula (II) to be a radical derived from a base B. According to this embodiment of the invention, a compound of formula (XIV) may be prepared directly by reaction of a thiirane of formula (III) in which the absolute configuration is (S), with a compound of formula (IV) in which E is a radical derived from a nucleic acid base B.

The compound of formula (IV) where E is a radical derived from a nucleoside purine or pyrimidine base can be formed by reaction of the base B with an α-halo acetate ester, for example an aryl, alkyl or cycloalkyl ester, under appropriate conditions, for example potassium carbonate in tetrahydrofuran.

According to a further aspect, the present invention provides the use of a compound of formula (II), in particular the mixtures of diastereomers of formulae (IIA) and (IIC) and of the enantiomers of formulae (IIB) and (IID), and the use of related compounds of formulae (IX) and (XIII), all as described above, as intermediates in the synthesis of 3′- or 4′-thionucleosides.

Compounds of formulae (II), (IX), (IX)L, especially those in substantially homochiral form, for example (IIA), (IIA)L, (IIB), (XII) and (X) are novel compounds and represent further features of the present invention.

The final stage in the production of the 4′-thionucleoside will generally be removal of the protecting group $P^1$. This group can be removed using conventional conditions such as boron tribromide cleavage ($P^1$ = $CH_2$Aryl), hydrolysis ($P^1$ = tetrahydropyran-2-yl) or fluoride ion cleavage ($P^1$ = silyl).

The optionally substituted purine and pyrimidine bases used in the formation of the 4′-thionucleosides are generally known compounds and are either commercially available or can be prepared by known methods.

The invention is illustrated further by the following preparation and examples in which the following experimental procedures were adopted. Reagents (including chiral glycidols) were purchased from the Aldrich Chemical Company, Gillingham, Dorset, U.K., with the exception of 4-dimethylaminopyridine and tetrabutylammonium fluoride on silica which were purchased from Fluka, Glossop, U.K. and N-Fluoro, N-chloromethyl triethylenediamine bis (tetrafluoroborate) (Selectfluor (Trademark)) which was obtained from Air Products and Chemicals plc, Walton-on-Thames, U.K.. Tetrahydrofuran (THF) was dried and distilled over lithium aluminium hydride, acetonitrile was similarly treated over calcium hydride; all other solvents were stored over molecular sieves. The term "petrol" refers to the distillate collected at between 40 and 60°C. Organic solutions were dried over anhydrous magnesium sulphate.

Preparation 1

(R)-(Tertbutyldiphenylsilyloxy-methyl)-thiirane

(i) (S)-Tertbutyldiphenylsilyl glycidol

To a solution of tert-butylchlorodiphenyl silane (19.5 g, 70.0 mmol), imidazole (4.82 g, 70.9 mmol), and 4-dimethylamino pyridine (0.41 g, 3.3 mmol) in dichloromethane (150 ml), cooled to 0°C under nitrogen, was added S-(-)-glycidol (5g) in a dropwise manner in 40 ml of dichloromethane. Reaction was complete after 2.5 h. The solution was washed with water, a back extraction combined, then washed with iced 1N HCl (x2), water and brine. After drying, the solution was evaporated and treated directly with the next reagent.

$^1$H NMR spectrum ($CDCl_3$) $\delta_H$, 1.05 (9H, s, $CMe_3$), 2.60 and 2.75 (ea 1H, dd, H-3), 3.10 (1H, m, H-2), 3.70 and 3.85 (ea 1H, dd, H-1), 7.40 to 7.70 (10H, m, Ar-H).

(ii) (R)-(Tertbutyldiphenylsilyloxy-methyl)-thiirane

The crude (S)-silyl glycidol from Preparation 1(i) (67.5 mmol) was dissolved in 300 ml of methanol to which was added thiourea (5.13 g, 67.5 mmol) in one portion. The solution was stirred for 14h, after which the solvent was evaporated. The residue was taken up in ether and washed with water (x2) and brine, then dried and evaporated. The residue was purified on a flash column eluted with neat petrol, to give the pure thiirane as a mobile oil.

$^1$H NMR spectrum ($CDCl_3$) $\delta_H$, 1.05 (9H, s, $CMe_3$), 2.10 and 2.45 (ea 1H, dd, H-3), 3.05 (1H, m, H-2), 3.55 and 3.95 (ea 1H, dd, H-1), 7.40 to 7.70 (10H, m, Ar-H).
CD Spectrum (hexane) 261 (-6.9)nm.

Example 1

4-(S)-(Tertbutyldiphenylsilyloxy-methyl)-4-thio-butanolactone

(i) 2-Carboxymethyl-4-(S)-(tertbutyldiphenylsilyloxymethyl)-4-thiobutanolactone

Dimethyl malonate (6.6 g. 50 mmol) in 75 ml of dry THF, was added dropwise to a solution of sodium bis(trimethyldisilyl)amide (50.1 mmol) in 250 ml of THF at room temperature. On completion of the addition, the thiirane from Preparation 1(ii) (13.7 g, 41.8 mmol) was added in one portion in 400 ml of THF, and the solution was refluxed for 80 h. On cooling, the solution volume was reduced and the residue partitioned between ether and saturated ammonium chloride. A second extraction was combined and washed with water and then brine before drying and evaporation. The residue was columned on silica, eluted with a gradient of 10-30% ether/petrol, which gave some unreacted thiirane plus the lactone.

$^1$H NMR spectrum ($CDCl_3$) $\delta_H$, 1.05 (9H, s, $CMe_3$), 2.30 to 2.70 (3H, m, H-2/3), 3.60 to 4.10 (3H, m, H-4/5), 3.75 (3H, s, OMe), 7.40 to 7.70 (10H, m, Ar-H).

Microanalysis, found: C, 64.42; H, 6.58: $C_{23}H_{28}O_4SSi$ requires C, 64.45; H, 6.58%.

(ii) 4-(S)-(Tertbutyldiphenylsilyloxy-methyl)-4-thio-butanolactone

The carboxymethyl lactone from Example 1(i) was dissolved in 75 ml of dimethylsulphoxide, to which were added 20 drops of brine. After 2 h at 170°C the reaction was complete. After cooling, the reaction solution was directly transferred onto a pre-packed silica column, and the title product eluted off with 30% ether in petrol.

$^1$H NMR spectrum ($CDCl_3$) $\delta_H$, 1.05 (9H, s, $CMe_3$), 2.05 to 2.55 (4H, m, H-2/3), 3.80 (2H, m, H-5), 4.05 (1H, m, H-4), 7.40 to 7.70 (10H, m, Ar-H).
CD Spectrum (EtOH) 231 (+1.2)nm.

Microanalysis, found: C, 67.73; H, 6.94: $C_{21}H_{26}O_2SSi$ requires C, 68.06; H 7.07%.

Example 2

4-(S)-(Tertbutyldiphenylsilyloxy-methyl)-2-(R)-phenylselenyl-4-thiobutanolactone

4-(S)-(Tertbutyldiphenylsilyloxy-methyl)-4-thio-butanolactone from Example 1(ii) (5.25 g, 14.2 mmol) in 45 ml of dry THF was added dropwise to a 1M solution of lithium hexamethyldisilazide (15.6 ml) in THF at -78°C

under nitrogen (this temperature was maintained throughout the reaction). This solution was stirred for 1 h on completion of the addition, then a single portion of trimethylsilylchloride (1.69 g, 15.6 mmol) was added and stirred for 2 h. At this time, phenylselenyl bromide (3.68g, 15.6 mmol) in 60 ml of THF was added slowly to the intermediate silylenol ether, and the reaction stirred for a further 1 h before it was allowed to warm to room temperature. The mixture was poured into water which was extracted with three portions of ether. These combined fractions were washed twice with brine, dried and evaporated. The resulting syrup was purified by elution of a flash column with a 0-10% ether/petrol gradient, affording the title compound.

$^1$H NMR spectrum ($CDCl_3$) $\delta_H$, 1.05 (9H, s, $CMe_3$), 2.35 (2H, m, H-3), 3.75 to 3.85 (4H, m, H-2/4/5), 7.30 to 7.65 (l5H, m, Ar-H).

Example 3

1-O-Acetyl-5-O-(tertbutyldiphenylsilyl)-2,3-dideoxy-2-phenylselenyl-4-thio-α/β-D-ribofuranose

4-(S)-(Tertbutyldiphenylsilyloxy-methyl)-2-(R)-phenylselenyl-4-thiobutanolactone from Example 2 (7.5 mmol) was reduced by diisobutylaluminiumhydride (7.9 mmol) in 100 ml dry toluene at -78°C over 3 h, after which the reaction was quenched with 100 ml of saturated ammonium chloride and vigorously stirred for 1 h. After filtering through a Hyflo (Trademark) pad, the organic layer was separated and washed twice with brine, dried and evaporated. The crude lactol was dissolved in 200 ml of dichloromethane and treated with 4-dimethylamino pyridine (1.0 g, 8.25 mmol) and acetic anhydride (0.84 g 8.25 mmol). Reaction was complete in 2 h at room temperature, then the solution was washed sequentially with water, copper sulphate, water and then brine, dried and evaporated. Purification was achieved by an appropriate gradient of ether/petrol on a flash column.

$^1$H NMR spectrum ($CDCl_3$) $\delta_H$, 1.00 and 1.05 (9H, 2s, $CMe_3$), 1.95 and 2.10 (3H, 2s, $CH_3CO$), 2.30 to 2.70 (2H, m, H-3), 3.45 to 3.95 (4H, m, H-2/4/5), 6.10 and 6.15 (1H, 2d, H-1), 7.40 to 7.70 (15H, m, Ar-H).

Example 4

5′-O-(Tertbutyldiphenylsilyl)-3′-deoxy-2′-α-phenylselenyl-4′-thio-β-D-thymidine

Thymine (4.7 mmol) was refluxed under nitrogen in hexamethyldisilazine (40 ml) containing 5 drops of trimethylsilyl chloride until it had all dissolved (about 3 h, a little ammonium sulphate may be added to assist dissolution). The solvent was then removed by distillation. 1-O-Acetyl-5-O-(tertbutyldiphenylsilyl)-2,3-dideoxy-2-phenylselenyl-4-thio -α/β-D-ribofuranose from Example 3 (2.35 mmol) was then added to the nitrogen blanketed bissilylated base as a solution in dry acetonitrile, and cooled to 0°C. Tin (IV) chloride (0.92 g, 3.5 mmol) was added to this mixture in a dropwise manner in a further 20 ml of acetonitrile and the reaction kept at 0°C for 30 min. 100 ml of both ethyl acetate and saturated sodium bicarbonate were then added, and the mixture vigorously stirred. This suspension was then filtered through Hyflo and the organic layer washed twice with brine, dried, and then evaporated and purified on a flash column eluted with 1:1 ether-petrol to give the title compound.

$^1$H NMR spectrum ($CDCl_3$) $\delta_H$, 1.05 (9H, s, $CMe_3$), 1.60 (3H, d, Me), 2.15 and 2.55 (ea 1H, m, H-3′), 3.70 (4H, m, H-2′/4′/5′), 6.30 (1H, d, H-1′), 7.05 (1H, d, H-6), 7.25 to 7.70 (15H, m, Ar-H), 7.75 (1H, br s, NH).

Infra red spectrum $\nu_{max}$ (KBr disc) 3 200, 1 689 $cm^{-1}$.

Example 5

5′-O-(Tertbutyldiphenylsilyl)-3′-deoxy-4′-thio-β-D-thymidine

5′-O-(Tertbutyldiphenylsilyl)-3′-deoxy-2′-α-phenylselenyl-4′-thio-β-D-thymidine (424 mg, 0.67 mmol) from Example 5 was dissolved in degassed benzene (15 ml) under argon, to which was added triethyl borane (solution in hexane, 1.1 equivalent). Tributyltin hydride (213 mg, 0.73 mmol) was added dropwise to this mixture in a further 6 ml of degassed benzene, and the reaction was complete in a further 30 min at room temperature. The reaction was quenched by addition of saturated ammonium chloride, the layers separated and the organic layer washed twice with brine, dried and evaporated. Purification of the residue on a flash column, eluted with 1:1 ether/petrol, gave the title compound.

$^1$H NMR spectrum ($CDCl_3$) $\delta_H$ 1.05 (9H, s, $CMe_3$), 1.80 (3H, d, Me), 1.90 to 2.40 (4H, m, H-2′/3′), 3.65 (1H, m, H-4′), 3.85 (2H, m, H-5′), 6.25 (1H, m, H-1′), 7.35 to 7.75 (10H, m, Ar-H), 7.50 (1H, m, H-6), 8.30 (1H, br s, NH).

Example 6

5'-O-(Tertbutyldiphenylsilyl)-2',3'-didehydro-3'-deoxy-4'-thio-β-D-thymidine

5'-O-(Tertbutyldiphenylsilyl)-3'-deoxy-2'-a-phenylselenyl-4'-thio-β-D-thymidine (1.05 g, 1.69 mmol) from Example 4 was dissolved in dry dichloromethane and cooled to -20°C under nitrogen. To this was added m-chloroperoxybenzoic acid (0.35 g, 1.2 eq) in one portion, and the temperature maintained during the course of the reaction (45 min). 5 Equivalents of pyridine were then added and the solution allowed to warm to room temperature over 1 hour. After dilution with dichloromethane the solution was washed successively with water, copper sulphate (x2), sodium bicarbonate (x2), water, and brine, before drying and evaporation. Purification of the residue on a flash column, eluted with 1:1 ether/petrol afforded the title compound.

$^1H$ NMR spectrum ($CDCl_3$) $\delta_H$, 1.05 (9H, s, $CMe_3$), 1.70 (3H, d, Me), 3.85 (2H, m, H-5'), 4.45 (1H, m, H-4'), 5.75 and 6.30 (ea 1H, m, H-2'/3'), 6.95 (1H, d, H-6), 7.05 (1H, dd, H-1'), 7.55 to 7.70 (10H, m, Ar-H), 8.05 (1H, br s, NH).

Microanalysis, found: C, 64.89; H, 6.30; N, 5.73 $C_{26}H_{30}O_3N_2SSi$ requires C, 65.24; H, 6.32; N, 5.85%.

Example 7

3'-Deoxy-4'-thio-β-D-thymidine

5'-O-(Tertbutyldiphenylsilyl)-3'-deoxy-4'-thio-β-D-thymidine from Example 5 was stirred in a THF suspension of tetrabutylammonium fluoride supported on silica gel (1.1 equivalent) for 1 hour at room temperature when completion was evident by tlc (neat ether). A little more silica was added, the solvent evaporated, and the suspension placed on a short silica pad. The product was eluted off with 5% ethanol in chloroform, and could be recrystallised from ethanol.

$^1H$ NMR spectrum ($CDCl_3$) $\delta_H$, 1.90 (3H, d, Me), 1.95 to 2.40 (4H, m, H-2'/3'), 3.70 (1H, m, H-4'), 3.95 (2H, m, H-5'), 6.30 (1H, m, H-1'), 7.85 (1H, m, H-6), 8.30 (1H, br s, NH).

Infra red spectrum $\nu_{max}$ (KBr disc) 3 422, 3 250, 1 686 $cm^{-1}$.

Example 8

2' ,3'-Didehydro-3'-deoxy-4'-thio-β-D-thymidine

From the title compound of Example 6 using the method of Example 5.

$^1H$ NMR spectrum ($CDCl_3$) $\delta_H$, 1.90 (3H, d, Me), 3.80 and 3.95 (ea 1H, m, H-5'), 4.45 (1H, m, H-4'), 5.70 and 6.25 (ea 1H, m, H-2'/3'), 7.00 (1H, dd, H-1'), 7.45 (1H, m, H-6), 7.95 (1H, br s, NH).
CD Spectrum (EtOH) 264 (-9.68)nm.

Example 9

1-O-Acetyl-5-O-(tertbutyldiphenylsilyl)-2,3-dideoxy-4-thio-α/β-D-ribofuranose

From the product of Example 1(ii) using the procedure of Example 3.

$^1H$ NMR spectrum ($CDCl_3$) $\delta_H$, 1.05(9H, s, $CMe_3$), 1.90 to 2.35 (4H, m, H-2/3), 2.00 and 2.05 (3H, 2s, $CH_3CO$), 3.45 to 3.85 (3H, m, H-4/5), 6.10 (1H, m, H-1), 7.40 to 7.70 (10H, m, Ar-H).

Infra red spectrum $\nu_{max}$ (neat film) 1 735 $cm^{-1}$.

Example 10

5'-O-(Tertbutyldiphenylsilyl)-2' ,3'-dideoxy-4'-thio-D-uridine

From 1-O-acetyl-5-O-(tertbutyldiphenylsilyl)-2,3-dideoxy-4-thio-α/βD-ribofuranose of Example 9 and the appropriate bissilylated base using the procedure of Example 4 followed by chromatography with an appropriate ether/petrol gradient.

β-anomer

$^1H$ NMR spectrum ($CDCl_3$) $\delta_H$, 1.05 (9H, s, $CMe_3$), 1.85 to 2.40 (4H, m, H-2'/3'), 3.70 (1H, m, H-4'), 3.90 (2H,

m, H-5′), 5.55 (1H, d, H-5), 6.25 (1H, m, H-1′), 7.35 to 7.75 (10H, m, Ar-H), 7.90 (1H, d, H-6), 9.50 (1H, br s, NH).

Infra red spectrum $\nu_{max}$ (KBr disc) 3 250, 1 688 $cm^{-1}$.

α-anomer

$^1$H NMR spectrum ($CDCl_3$) $\delta_H$, 1.05 (9H, s, $CMe_3$), 1.90 to 2.40 (4H, m, H-2′/3′), 3.65 (2H, m, H-5′), 3.85 (1H, m, H-4′), 5.80 (1H, d, H-5), 6.25 (1H, m, H-1′), 7.35 to 7.75 (10H, m, Ar-H), 7.80 (1H, d, H-6), 9.00 (1H, br s, NH).

Infra red spectrum $\nu_{max}$ (KBr disc) 3 250, 1 688 $cm^{-1}$.

Microanalysis, found: C, 64.59; H, 6.61; N, 5.91 $C_{25}H_{30}O_3N_2SSi$ requires C, 64.34; H, 6.48; N, 6.00%

Example 11

2′,3′-Dideoxy-4′-thio-β-D-uridine

From the β anomer product of Example 10 by the method of Example 7.

$^1$H NMR spectrum (DMSO-$d_6$) $\delta_H$, 1.80 to 2.30 (4H, m, H-2′/3′), 3.45 to 3.75 (3H, m, H-4′/5′), 5.10 (1H,br m, OH), 5.65 (1H, dd, H-5), 6.10 (1H, m, H-1′), 8.05 (1H, d, H-6), 11.30 (1H, br s, NH).

Infra red spectrum $\nu_{max}$ (KBr disc) 3 520, 3 250, and 1 680 $cm^{-1}$.

Example 12

5-O-Tertbutyldiphenylsilyl-2,3-didehydro-2,3-dideoxy-4-thio-D-ribonolactone

From 4-(S)-(tertbutyldiphenylsilyloxy-methyl)-2-(R)-phenylselenyl-4-thio-butanolactone of Example 2 by the method of Example 6.

$^1$H NMR spectrum ($CDCl_3$) $\delta_H$, 1.05 (9H, s, $CMe_3$), 3.90 (2H, m, H-5), 4.60 (1H, m, H-4), 6.30 (1H, dd, H-2), 7.30 to 7.65 (10H, m, Ar-H), 7.55 (1H, dd, H-3).

Example 13

5′-0-(Tertbutyldiphenylsilyl)-2′ ,3′-dideoxy-4′-thio-α/β-cytidine

The product of Example 9 (1.24g, 3.0 mmol), cytosine (502 mg, 4.5 mmol) and potassium nonafluorobutanesulphonate (3.65g, 10.8 mmol) were suspended together in dry acetonitrile (40 ml) under nitrogen. Hexamethyldisilazane (631 μl, 3 mmol) and trimethylsilyl chloride (1.75 ml, 13.8 mmol) were each added to this in one portion. The resulting mixture was stirred vigorously at room temperature for 15h, after which it was partitioned between dichloromethane and saturated aqueous sodium bicarbonate. A back extraction with dichloromethane and the organic fraction were combined and washed with brine then dried and evaporated. Purification by flash chromatography eluting with chloroform/methanol/aqueous ammonia (93:7:1) gave separation of the title anomers.

β anomer:

$^1$H NMR spectrum ($CDCl_3$) $\delta_H$, 1.05 (9H, s, $CMe_3$), 1.80-2.40 (4H, m, H-2′/3′), 3.65 (1H, m, H-4′), 3.85 (2H, m, H-5′), 5.45 (1H, d, H-5), 5.90 (2H, br, $NH_2$), 6.35 (1H, dd, H-1′), 7.40 (6H, m, Ar-H), 7.70 (4H, m, Ar-H), 8.05 (1H, d ,H-6).

α-anomer:

$^1$H NMR Spectrum ($CDCl_3$) $\delta_H$, 1.05 (9H, s, $CMe_3$), 1.80-2.40 (4H, m, H-2′/3′), 3.65 (2H, m, H-5′), 3.80 (1H, m, H-4′), 5.75 (1H, d, H-5), 5.90 (2H, br, $NH_2$), 6.30 (1H, dd, H-1′), 7.40 (6H, m, Ar-H), 7.65 (4H, m, Ar-H), 7.95 (1H, d, H-6).

Example 14

2′ ,3′-Dideoxy-4′-thio-β-D-cytidine

The β-anomer product of Example 13 (140 mg, 0.30 mmol) was dissolved in THF and treated according to the method of Example 7. Purification by elution from a short silica pad with chloroform/methanol/aqueous ammonia (93:7:1) and subsequently by preparative reverse-phase HPLC then freeze-drying gave the title product as a white lyopholyte.

$^1$H NMR spectrum (DMSO-$d_6$) $\delta_H$, 1.80-2.20 (4H, m, H-2′/3′), 3.50-3.75 (3H, m, H-4′/5′), 5.10 (1H, br s, OH), 5.85 (1H, d, H-5), 6.15 (1H, dd, H-1′), 7.30 and 7.70 (ea 1H, br s, -$NH_2$), 8.10 (1H, d, H-6).

Infra red spectrum $\nu_{max}$ (KBr disc) 3 337, 3 190, 1 718 and 1 645 $cm^{-1}$.

Example 15

4-(S)-Tertbutyldiphenylsilyloxy-methyl)-2-(R/S)-fluoro-4-thio-butanolactone

The product from Example 1(ii) (1.11 g,3.0 mmol) was stirred at -78°C under nitrogen with lithium hexamethyldisilazide (3.3 mmol) for one hour. Trimethylsilyl trifluoroacetate was added to this solution and the reaction stirred to room temperature for 2 hours. A solution of N-fluoro, N-chloromethyl triethylenediamine bis(tetrafluoroborate) in dry acetonitrile (30 ml) was added to this and the mixture stirred under nitrogen for 45 minutes. The resulting suspension was partitioned between diethyl ether and water, the water layer extracted twice with ether and the combined ether fractions washed with brine, dried and evaporated. Purification of the residue by flash chromatography eluting with 10% ether in petrol gave the title product mainly as the 2-(R)-fluoro diastereomer with traces of the corresponding 2-(S)-fluoro diastereomer.

2-(R)-Fluoro diastereomer:

$^1$H NMR spectrum ($CDCl_3$), $\delta_H$, 1.05 (9H, s, $CMe_3$), 2.45 (2H, m, H-3), 3.85 (2H, m, H-5), 4.05 (1H, m, H-4), 5.15 (1H, dt, H-2), 7.40 (6H, m, ArH), 7.65 (4H, m, ArH).

Infra-red spectrum $\nu_{max}$ (KBr disc) 1 718 and 1 703 $cm^{-1}$.

Microanalysis, found: C,64.99; H,6.63%; $C_{21}H_{25}O_2SSiF$ requires C,64.91; H,6.49%.

2-(S)-Fluoro diastereomer:

$^1$H NMR Spectrum ($CDCl_3$) $\delta_H$, 1.05 (9H, s, $CMe_3$), 2.1-2.65 (2H, m, H-3), 3.85 (3H, m, H-4/5), 5.05 (1H, ddd, H-2), 7.40 (6H, m, Ar-H), 7.65 (4H, m, Ar-H).

Example 16

5-0-(Tertbutyldiphenylsilyl)-3-deoxy-4-thio-D-ribonolactone

The product from Example 1(ii) (315mg, 0.85mmol) was dissolved in dry toluene (15ml) and cooled to -78°C under nitrogen. Potassium hexamethyl disilazide (0.5M in toluene, 0.94mmol) was added in one portion and stirred at -78°C for one hour. (±)-Trans-2-(phenylsulphonyl)-3-phenyloxaziridine in dry toluene (5ml) was added to this in one portion and the reaction stirred at -78°C for 3 hours then at room temperature overnight. The crude mixture was poured into water, extracted twice with toluene and the combined extracts washed twice with brine, dried and evaporated. Purification by flash chromatography eluting with 1:1 diethyl ether/petrol afforded the title product.

$^1$H NMR ($CDCl_3$) $\delta_H$, 1.05 (9H, s, $CMe_3$), 2.20 and 2.60 (ea 1H, m, H-3), 2.75 (1H, br d, OH), 3.85 (3H, m, H-4/5), 4.45 (1H, m, H-2), 7.40 (6H, m, Ar-H), 7.65 (4H, m, Ar-H).

Infra-red Spectrum $\nu_{max}$ (thin film) 3 400 and 1 695 $cm^{-1}$.

Example 17

2-(S)-Bromo-4-(R)-(tertbutyldiphenylsilyloxymethyl)-4-thio-butanolactone

4-(R)-(Tertbutyldiphenylsilyloxymethyl)-4-thio-butanolactone (1.11g, 3.0mmol) was converted to the corresponding silyl enol ether according to the method of Example 2. The resulting solution was warmed to room

temperature and the solvent evaporated to give a crude oil which was kept under nitrogen then dissolved in chloroform (30ml) and cooled to -10°C under nitrogen. Bromine (528 mg, 1.1eq) in chloroform (10ml) was added and the solution stirred to room temperature for 14 hours. The solution was then washed with water, back extracted and washed twice with brine, dried and evaporated. Purification of the residue by flash chromatography eluting with 10% ether in petrol afforded the title product as an oil which crystallised on standing.

$^1H$ NMR Spectrum ($CDCl_3$) $\delta_H$, 1.05 (9H, s, $CMe_3$), 2.45 (2H, m, H-3), 3.85 (2H, m, H-5), 4.25 (1H, m, H-4), 4.55 (1H, dd, H-2), 7.40 (6H, m, Ar-H), 7.65 (4H, m, Ar-H).

Microanalysis found C, 56.16; H, 5.71%. $C_{21}H_{25}Br0_2SSi$ requires C,56.11; H,5.61%

Example 18

4-(R)-(Tertbutyldiphenylsilyloxymethyl)-2-(S)-(6-chloropurin-9-yl)-4-thio-butanolactone

To the product of Example 17 (360mg, 0.8mmol) in dry DMF (15ml) under nitrogen were added 6-chloropurine (248mg, 1.6mmol), 18-crown-6 (212mg, 0.8mmol) and potassium carbonate (221mg, 1.60mmol). The mixture was heated to 75°C for 3 hours then poured into water. The organic layer was washed four times with water and once with brine then dried and evaporated. Purification of the residue by flash chromatography eluting with diethyl ether gave the title compound and its N-7 isomer in a ratio of 3:1.

N-9 Purinyl substituted compound:

$^1H$ NMR Spectrum ($CDCl_3$) $\delta_H$, 1.05 (9H, s, $CMe_3$), 2.60 and 2.85 (ea 1H, m, H-3), 3.95 (2H, m, H-5), 4.15 (1H, m, H-4), 5.45 (1H, dd, H-2), 7.40 (6H, m, Ar-H), 7.65 (4H, m, Ar-H), 8.05 (1H, s, H-8p), 8.70 (1H, s, H-2p).

Microanalysis found C, 60.00; H, 5.29: N, 10.38%: $C_{26}H_{27}N_4SO_2SiCl$ requires C,59.70; H,5.20; N,10.71%.

N-7 Purinyl substituted compound:

$^1H$ NMR Spectrum ($CDCl_3$) $\delta_H$, 1.05 (9H, s, $CMe_3$), 2.85 and 3.10 (ea 1H, m, H-3), 4.00 (3H, m, H-4/5), 5.65 (1H, dd, H-2), 7.40 (6H, m, Ar-H), 7.65 (4H, m, Ar-H), 7.90 (1H, s, H-8p), 8.70 (1H, s, H-2p).

Example 19

9-[5′-O-(Tertbutyldiphenylsilyl)-2′ ,3′-dideoxy-2′-phenylseleno-4′-thio-β-D-ribofuranosyl]-6-chloropurine

6-Chloropurine (1.24g, 8.0mmol) was refluxed with bis(trimethylsilyl)-acetamide in dry toluene (50ml) for 45 minutes. The solvent was evaporated and the residue taken up in dry acetonitrile (75ml) containing the product from Example 3 (2.28g, 4.0mmol). The mixture was cooled to 0°C and a solution of diethyl aluminium chloride (4mmol) in dry acetonitrile (30ml) was added dropwise. The resulting mixture was stirred at 0°C for one hour then at room temperature for 14 hours. Aqueous sodium bicarbonate and ethyl acetate were added to give a suspension which was filtered through Hyflo. The organic layer was washed twice with brine, then dried and evaporated. Purification of the residue by flash chromatography, eluting with 1:1 diethyl ether/petrol gave the title product.

$^1H$ NMR Spectrum ($CDCl_3$) $\delta_H$, 1.05 (9H, s, $CMe_3$), 2.25 and 2.60 (ea 1H, m, H-3′), 3.80 (1H, m, H-4′), 3.90 and 4.05 (ea 1H, dd, H-5′), 4.25 (1H, m, H-2′), 6.15 (1H, d, H-1′), 7.10-7.50 (11H, m, Ar-H), 7.70 (4H, m, Ar-H), 8.10 (1H, s, H-8), 8.55 (1H, s, H-2).

Example 20

9-[5′-0-(Tertbutyldiphenylsilyl)-2′ ,3′-didehydro-2′ -3′-dideoxy-4′-thio-β-D-ribofuranosyl]-6-chloropurine

The title product was obtained by treatment of the product from Example 19 according to the method of Example 6.

$^1H$ NMR Spectrum (DMSO-$d_6$) $\delta_H$, 1.05 (9H, s, $CMe_3$), 3.10 and 3.90 (ea 1H, dd, H-5′), 4.60 (1H, m, H-4′), 6.45 and 6.70 (ea 1H, dt, H-2′/3′), 7.35 (6H, m, Ar-H), 7.60 (4H, m, Ar-H), 8.50 (1H, s, H-8), 8.70 (1H, s, H-2).

## Claims

1. A process for the preparation of a compound of formula (II)

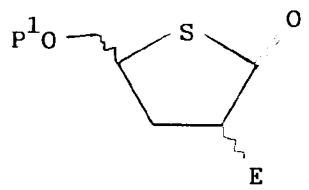

(II)

wherein $P^1$ is a protecting group or hydrogen and E is an anion stabilising group;
which comprises reacting a compound of formula (III)

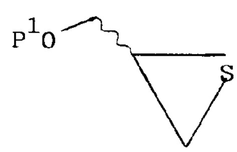

(III)

wherein $P^1$ is as defined above,
with a compound of formula (IV)

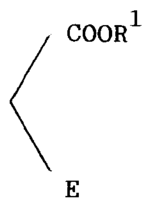

(IV)

wherein E is as defined above and $R^1$ is $C_{1-6}$ alkyl, $C_{1-6}$ cycloalkyl or aryl,
and optionally, subsequent removal of any protecting group $P^1$.

2. A process according to Claim 1 for the preparation of a compound of formula (II) having the 4(S)-configuration, wherein the thiirane of formula (III) has the 4(S)-configuration.

3. A process according to Claim 1 for the preparation of a compound of formula (II) having the 4(R)-configuration, wherein the thiirane of formula (III) has the 4(R)-configuration.

4. A process according to Claims 1, 2, or 3 wherein $P^1$ is hydrogen or a silyl group and E is a carboxyalkyl group.

5. A process according to Claims 1, 2, or 3 wherein $P^1$ is hydrogen, tertbutyldiphenylsilyl or tertbutyldimethylsilyl and E is carboxymethyl.

6. A process according to any one of Claims 1-5 in which the resulting compound of formula (II) is converted to a compound of formula (IX)

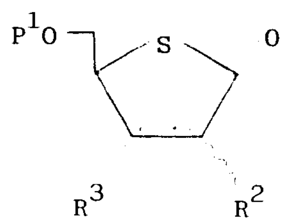

(IX)

wherein $R^2$ and $R^3$ are both hydrogen.

7. A process according to Claim 6 wherein the compound of formula (IX) produced is converted to a compound of formula (IX) wherein $R^2$ is phenylsulphenyl, phenylsulphinyl, phenylsulphonyl or phenylselenyl,

which comprises,
(i) formation of the corresponding enolate by treatment with base or formation of a corresponding silenol ether by treatment with a base and a silyl halide, and then
(ii) treatment with a phenylsulphenyl, phenylsulphinyl, phenylsulphonyl or phenylselenyl halide.

**8.** A process according to Claim 7 wherein the product of formula (IX) in which $R^2$ is phenylselenyl is converted into a compound of formula (IX) in which $R^2$ and $R^3$ together form a double bond,
which comprises selective oxidation of the selenium ligand.

**9.** A process according to Claim 6 wherein the product of formula (IX) is converted to a compound of formula (IX) in which $R^2$ is fluorine, bromine or hydroxyl,
which comprises,
(i) formation of the corresponding enolate by treatment with base or formation a corresponding silenol ether by treatment with base and a silyl halide, and then
(ii) treatment with an electrophilic fluorinating agent, brominating agent or hydroxylating agent.

**10.** A process according to Claim 9 wherein the product of formula (IX) is obtained with substituent $R^2$ in substantially α-configuration.

**11.** A process according to Claim 9 or 10 wherein the product of formula (IX) is further reacted to eliminate H-$R^2$ to give a product of formula (IX) in which $R^2$ and $R^3$ together form a double bond.

**12.** A process according to either of Claims 8 or 11 wherein the product of formula (IX) in which $R^2$ and $R^3$ together form a double bond is further reacted to introduce oxygen-based functionality into the 2′ and 3′ positions.

**13.** A process according to Claim 10 wherein the product of formula (IX) in which $R^2$ is α-hydroxy is further treated with diethylaminosulphurtrifluoride to provide a product of formula (IX) in which $R^2$ is β-fluorine.

**14.** A process according to Claim 7 wherein the product of formula (IX) is obtained with substituent $R^2$ in substantially α-configuration ie. the product is a compound of formula (IIB)

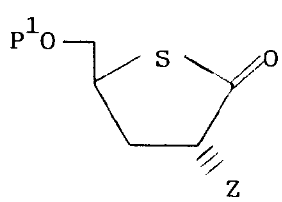

(IIB)

wherein $P^1$ is as defined above and Z is a directing group.

**15.** A process according to any one of Claims 1, 2, 4-14 wherein the product of formula (II), (IX) or (IIB) is further reacted to form a compound of formula (IIA)L, (IX)L or (X) respectively

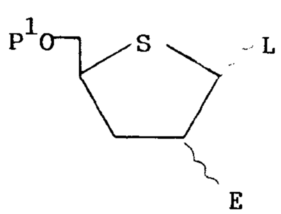

(IIA)L

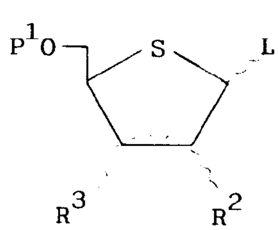

(IX)L

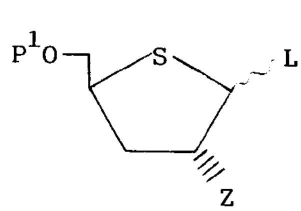

(X)

wherein $P^1$, E, $R^2$ and Z are as defined above and L is a leaving group;
which comprises,
(i) reduction of the carbonyl group to a hydroxyl group, then
(ii) treatment of the hydroxyl group to give a leaving group.

**16.** A process according to Claim 15 wherein the product of formula (IIA)L, (IX)L or (X) is further reacted (with a purine or pyrimidine base in optionally protected form) to provide a 4′-thionucleoside of formula (VIA), (VI) or (XI)

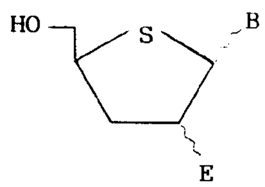

(VIA)

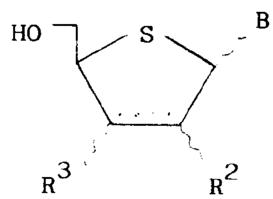

(VI)

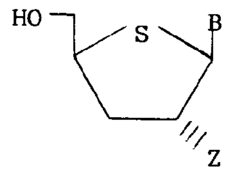

(XI)

wherein E, $R^2$, $R^3$ and Z are as defined above and B is a purine or pyrimidine base,

which comprises,

(i) treatment with a purine or pyrimidine base in optionally protected form,

(ii) optional removal of any protecting groups,

(iii) optional separation of the α and β anomers of products (VIA) and (VI)

**17.** A process according to Claim 3 wherein the product of formula (II) is further treated to form a 3′ thionucleoside of formula (XIV)

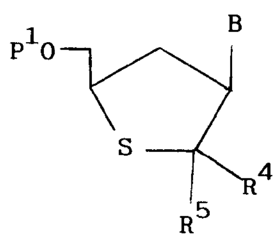

(XIV)

wherein

$P^1$ and B are as defined above and

$R^4$ and $R^5$ both represent hydrogen;

$R^4$ and $R^5$ taken together represent =O;

$R^4$ is hydrogen and $R^5$ is -OH or protected OH; or

$R^4$ is hydroxyl or protected OH and $R^5$ is H.

which comprises,

(i) formation of a compound of formula (IID)

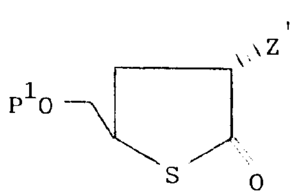

(IID)

where $P^1$ is as defined above and Z′ is a leaving group

(ii) treatment with an optionally protected purine or pyrimidine base in basic conditions

(iii) optional reduction of the carbonyl group using standard procedures

(iv) optional removal of any protecting groups

**18.** A compound of formula (IIB) or (IID)

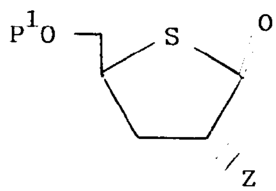

(IIB)

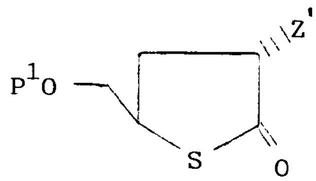

(IID)

wherein $P^1$ is hydrogen or a protecting group, Z is a directing group, and Z′ is a leaving group.

**19.** A compound of formula (IIA) or (IIC)

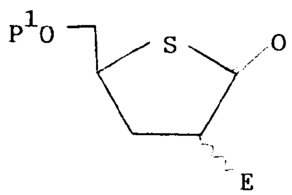

(IIA)

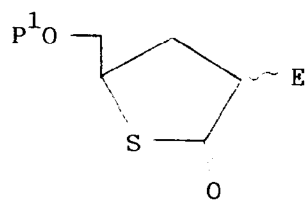

(IIC)

wherein $P^1$ is hydrogen or a hydroxyl protecting group, and E is an anion stabilising group

**20.** A compound of formula (IX)

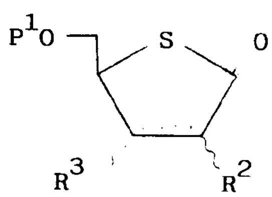

(IX)

wherein $P^1$ is H or a protecting group,
$R^2$ is H and $R^3$ is H, F, OH, $CH_2OH$ or $N_3$;
$R^2$ is OH or $CH_2OH$ and $R^3$ is H, OH or F;
$R^2$ is F and $R^3$ is H or OH; or
$R^2$ and $R^3$ together form a double bond
in the form of a single diastereomer.

**21.** A compound of formula (IIA)L, (IX)L or (X)

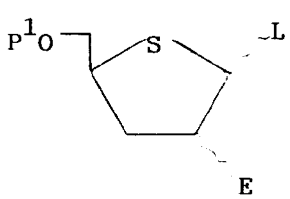

(IIA)L

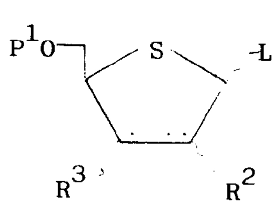

(IX)L

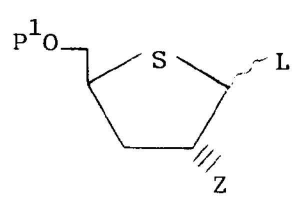

(X)

wherein $P^1$, E, $R^2$, $R^3$, and Z are as defined above and L is a leaving group.

22. A compound according to any one of Claims 18, 19, 20 or 21 wherein $P^1$ is hydrogen, tertbutyldiphenylsilyl or tertbutyldimethylsilyl, Z is phenylselenyl, Z′ is bromo or acetoxy, E is $-CO_2Me$ and L is acetoxy.

23. Use of a compound of formula (IIB) according to Claim 18 in the synthesis of 4′-thionucleosides of formula (XI)

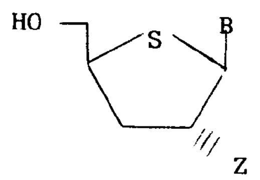

(XI)

wherein B and Z are as defined above

24. Use of a compound of formula (IID) according to Claim 18 in the synthesis of 3′-thionucleosides of formula (XIV) as defined in Claim 19.

25. Use of a compound of formula (IIA), (IIC), or (IX) as an intermediate in the synthesis of 3′ or 4′-thionucleosides.